(19) Europäisches Patentamt
European Patent Office
Office européen des brevets

(11) EP 1 950 232 A1

(12) EUROPEAN PATENT APPLICATION

(43) Date of publication:
**30.07.2008 Bulletin 2008/31**

(21) Application number: **07101223.1**

(22) Date of filing: **26.01.2007**

(51) Int Cl.:
***C08F 291/08*** (2006.01) ***C08F 265/04*** (2006.01)
***C08F 2/38*** (2006.01) *A61K 9/00* (2006.01)
*A61L 27/14* (2006.01) *A61L 31/04* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Polymers Australia PTY Limited**
**Notting Hill, VIC 3168 (AU)**

(72) Inventors:
- **Barner-Kowollik, Christopher**
  **Kensington, New South Wales 2033 (AU)**
- **LeFay, Catherine**
  **13011 Marseille (FR)**
- **Le Hellaye, Maud**
  **Sutherland, New South Wales 2232 (AU)**
- **Davies, Thomas Paul**
  **Randwick, New South Wales 2031 (AU)**
- **Stenzel, Martina H.**
  **Kirrawee, New South Wales 2232 (AU)**

(74) Representative: **Hübner, Gerd**
**Rau, Schneck & Hübner**
**Patentanwälte**
**Königstrasse 2**
**90402 Nürnberg (DE)**

Remarks:
claims 11-17, 19 deemed abandoned

# (54) Process for the preparation of graft copolymers by Reversible Addition Fragmentation Chain Transfer (RAFT) and Ring Opening Polymerisation (ROP)

(57) A combined RAFT/ROP process for the preparation of a graft co-polymer having a backbone built-up from repeating units derived by polymerization of free-radically polymerizable monomers comprising vinylic monomers, and polyester side chains grafted to said backbone derived by ring-opening polymerisation of cyclic ester compounds; said process comprising reacting a mixture of
(a) one or more vinylic monomers, optionally in combination with other monomers susceptible to free-radical polymerization, wherein at least one of said vinylic monomers comprises a (free) hydroxyl group;
(b) a thiocarbonylthio compound as chain transfer agent;
(c) one or more cyclic esters, which is/are susceptible to a ring-opening polymerization; and
(d) a compound effective for initiating the ring-opening polymerization of the one or more cyclic esters;
and reacting said mixture in an inert solvent at a temperature of more than 90°C under exclusion of oxygen to form the graft copolymer.

Fig.3 . Comparison of the RI and UV traces of the purified PHEMA-*g*-PCL copolymer (size exclusion chromatography in THF, 25 °C, 1 mL.min$^{-1}$, UV chromatogram recorded at 290 nm).

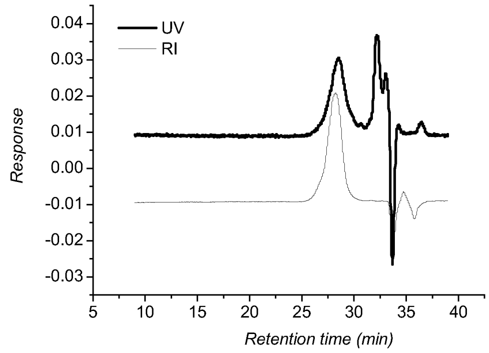


EP 1 950 232 A1

## Description

The present invention relates to a novel process for preparing graft-polymers, in particular to a process for preparing graft polymers wherein the grafting component is polymerizable by ring opening polymerization (ROP), novel compositions for the preparation of graft polymers and the use of certain graft polymers manufactured by said process.

In recent years so-called "living polymerizations" have become increasingly popular and important. According to IUPAC terminology "living polymerizations" are chain polymerizations from which irreversible chain transfer and irreversible chain termination (deactivation) are absent. Living polymerizations provide, in particular, the possibility to produce polymers with a predictable molecular weight and very narrow distribution in molecular weight (PDI e.g. <1.2). Living free-radical type polymerisations have been developed from the mid-ninties and include in particular the so-called "atom transfer radical polymerization" (ATRP) and the so-called "reversible addition-fragmentation transfer" polymerization (frequently and herein abbreviated as RAFT).

ATRP involves the use of transition metal compounds and thus provided products contaminated with metal atoms, a disadvantage for certain applications, in particular in the field of life sciences.

RAFT polymerization is performed by adding a RAFT agent, usually a dithiocarboxylate compound, to a conventional free-radical polymerization system. RAFT polymerizations have been used e.g. for the controlled polymerization of styrenes, (meth)acrylates, acrylamides and vinyl acetate derivatives. Typical polymerization conditions are 60°C and more and polymerization times of 2hrs and more. The general mechanism is as follows:

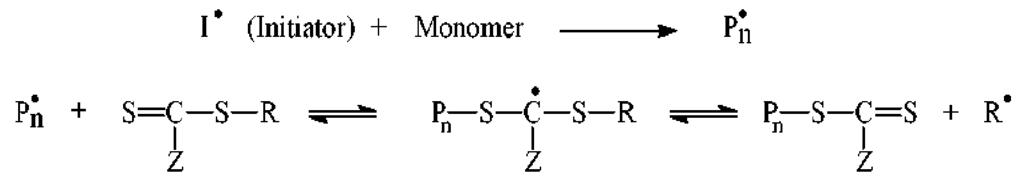


1 2 3

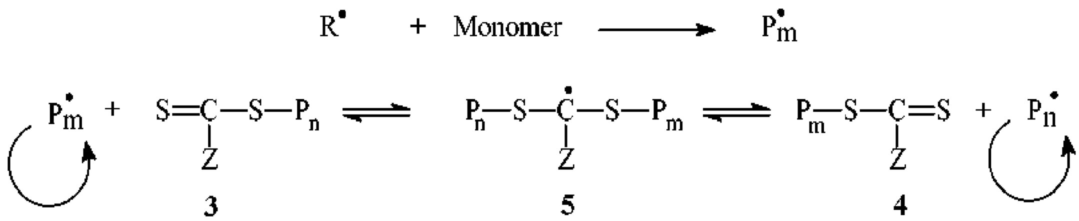


3 5 4

Addition of a propagating radical to the thiocarbonylthio (dithiocarboxylate) compound 1, gives the adduct radical 2, which fragments into a polymeric dithiocarboxylate compound 3 and a new radical R·. This radical re-initiates a polymerization with the formation of a new propagating radical that reacts again with 1. Subsequent addition fragmentation steps lead to an equilibrium between the propagating radicals $P_n$ and $P_m$ and the dormant thiocarbonylthio compound through the intermediate radical 5. As a result, the over all reaction can be described by:

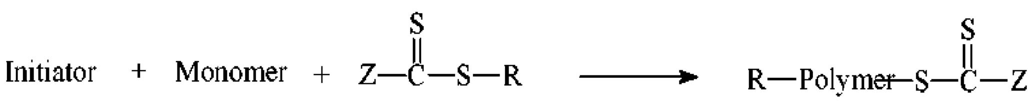


The species 1, 3, and 4 should preferably have high chain transfer constants. The Z-group should activate the C=S bond towards radical addition (aryl or alkyl) whereas the R-group should be a good free radical leaving group (e.g.

cumyl or cyanoisopropyl) and also be effective as initiator.

Graft polymerization offers an effective approach to incorporate desired properties into a polymer having a given backbone. For example, grafting e.g. a (meth)acrylate polymer or copolymer with e.g. poly (ε-caprolactone) and/or polylactide groups can impart improved environmental compatibleness, biocompatability and/or biodegradeability to these polymers. Such polymers are known to be valuble base materials in many biomedical and pharmacological areas, and can be used e.g. as scaffolds, meshes or sutures.

Graft poylmers like the aforementioned have already been manufactured by processes involving RAFT polymerisation of the backbone of the graft polymer. X. XU and J. HUANG, "Synthesis and Characterization of Well-Defined Poly(2-hydroxyethyl methacrylate-co-styrene)-graft-poly(ε-caprolactone) by Sequential Controlled Polymerization"; Journal of Polymer Science Part A: Polymer Chemistry, Vol. 42, 5523.5529 (2004) describe the preparation of poly(2-hydroxyethyl methacrylate-*co*-styrene)-graft-poly(ε-caprolactone) in a two stage process, the first stage being a RAFT step for forming the hydroxyethyl methacrylate-styrene co-polymer backbone, the hydroxyethyl methacrylate units of which are then reacted in a "ring opening polymerization" (frequently and herein abbreviated as ROP) step with ε-caprolactone to form the desired graft polymer.

The present invention aims at improving the process for manufacturing graft polymers like the aforementioned. It has been found that the RAFT step and the ROP step can be readily carried out as a one-stage reaction immediately forming the desired graft polymer, i.e. that these entirely different polymerization reactions can be carried out in the same pot and at the same time without any intermediate actions being required.

Subject of the present invention is therefore a combined RAFT/ROP process for the preparation of a graft *co*-polymer having a backbone built-up from repeating units derived by polymerization of free-radically polymerizable monomers comprising vinylic monomers, and polyester side chains grafted to said backbone derived by ring-opening polymerisation of cyclic ester compounds; said process comprising reacting a mixture of

(a) one or more vinylic monomers, optionally in combination with other monomers susceptible to free-radical polymerization, wherein at least one of said vinylic monomers comprises a (free) hydroxyl group;
(b) a thiocarbonylthio compound as chain transfer agent;
(c) one or more cyclic esters, which is/are susceptible to a ring-opening polymerization; and
(d) a compound effective for initiating the ring-opening polymerization of the one or more cyclic esters;

and reacting said mixture in an inert solvent at a temperature of more than 90°C under exclusion of oxygen to form the graft copolymer.

In a further aspect the present invention relates to a novel composition useful for making polyester-grafted polymers of the monomers (a), said composition comprising

(a) one or more vinylic monomers, optionally in combination with other monomers susceptible to free-radical polymerization, wherein at least one of said vinylic monomers comprises a (free) hydroxyl group;
(b) a thiocarbonylthio compound as chain transfer agent;
(c) one or more cyclic esters, which is/are susceptible to a ring-opening polymerization; and
(d) a compound effective for initiating the ring-opening polymerization of the one or more cyclic esters.

The process according to this invention is concurrently performed in one pot. It is not necessary to isolate any intermediate product, e.g. the parent hydroxy-functionalized polymer consisiting of the polymerized vinylic monomers.

The RAFT polymerization of one or more vinylic monomers *per se* is known in the art and, for example, described in detail in WO-A-98/01478, the disclosure of which is included herein by reference. As mentioned already, a RAFT polymerization system is basically a conventional free-radical polymerization system which additionally comprises a specific chain transfer agent, the "RAFT agent", usually a thiocarbonyl-thio compound, as described more particularly in WO-A-98/01478. For the purposes of the present invention said RAFT agent is preferably a compound of the following formula

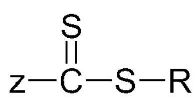

wherein

Z is selected from hydrogen, fluorine, chlorine, optionally substituted alkyl, optionally substituted aryl. optionally substituted heterocyclyl, optionally substituted alkylthio, - COOR", -COOH, $-O_2CR''$, $-CONR''_2$), -CN, $-P(=O)OR''_2$, and - P(=0)

R"$_2$]-, wherein R" is selected from optionally substituted $C_1$-$C_{18}$ alkyl, $C_2$-$C_{18}$ alkenyl, aryl, heterocyclyl, aralkyl, alkaryl wherein the substituents are independently selected from the group that consists of epoxy, hydroxy, alkoxy, acyl, acyloxy. carboxy (and salts), sulfonic acid (and salts), alkoxy- or aryloxy-carbonyl, isocyanato, cyano, silyl. halo, and dialkylamino; and

R is selected from optionally substituted alkyl; an optionally substituted saturated, unsaturated or aromatic carbocyclic or heterocyclic ring; optionally substituted alkylthio; optionally substituted alkoxy; optionally substituted dialkylamino.

For the purposes of this application and where not otherwise stated, alkyl means preferably $C_1$-$C_{18}$ alkyl, more preferably $C_1$-$C_6$ alkyl; aryl and heterocyclyl mean preferably corresponding groups having 5 to 14 ring atoms. Complex group notations like alkoxy, aryloxy, aralyl or alkaryl etc. are preferably meant to refer to residues including the respective molecular sub-parts as defined above.

Preferred thiocarbonylthio compounds useful for the purposes of the present invention include, for example, dithiobenzoic acid benzyl ester; dithiobenzoic acid 1-phenyl-ethyl ester; dithiobenzoic acid 1-methyl-1-phenyl-ethyl ester; acetic acid 1-thiobenzoylsulfanyl-ethyl ester; dithiobenzoic acid 1-(4-methoxyphenyl)-ethyl ester; thiobenzoylsulfanyl-acetic acid ethyl ester; 2-methyl-2-thiobenzoylsulfanyl-propionic acid ethyl ester; dithiobenzoic acid tert.-butyl ester; dithiobenzoic acid cyano-dimethyl-methyl ester (=2-(2-cyanopropyl) dithiobenzoate); dithiobenzoic acid 1,1,3,3-tetramethyl-butyl ester; dithiobenzoic acid 1-(4-chloro-phenyl)-1-methyl-ethyl ester; 4-chloro-dithiobenzoic acid 1-methyl-1-phenyl-ethyl ester; naphthalen-1-carbodithionic acid 1-methyl-1-phenyl-ethyl ester and 4-cyano-4-methyl-4-thiobenzoylsulfanyl-butyric acid. 2-(2-cyanopropyl) dithiobenzoate) is mostly preferred.

The quantity of chain transfer agent is adapted to the target backbone polymerization degree, DP, the initial concentration of RAFT agent, $[RAFT]_0$, and the DP being related by the relation (1):

$$Dp = \frac{[M]_0 - [M]_t}{[RAFT]_0} \qquad (1)$$

where $[M]_0$-$[M]_t$ is the backbone monomer consumed.

The process of the present invention can be applied to any monomers or monomer combination susceptible to free-radical polymerization as far as at least one vinylic monomer is comprised among said monomers which comprises a hydroxyl group as toehold for the graft component of the co-polymer.

Suitable free-radically polymerizable monomers for application in the present invention include e.g. vinylic monomers, preferably selected from the monomers of the following formula

$CH_2$=CUV

wherein

U is selected from hydrogen, halogen, optionally substituted $C_1$-$C_4$alkyl wherein the substituents are independently selected from hydroxy, $C_1$-$C_{18}$ alkoxy, aryloxy, carboxy, -$O_2$CR", $C_1$-$C_{18}$ alkoxycarbonyl and aryloxycarbonyl; and

V is selected from hydrogen, R", $CO_2H$, $CO_2$R", COR" , CN, $CONH_2$, CONHR", CONR"$_2$, $O_2$CR", OR" and halogen, wherein R" has the same meaning as already described above in the context of the RAFT agents.

Other free-radically polymerizable monomers that can be present are e.g. maleic anhydride, N-alkylmaleiimide or dialkyl fumarate.

Particularly preferred vinylic monomers are e.g. methyl methacrylate, ethyl methacrylate, propyl methacrylate (all isomers), butyl methacrylate (all isomers), 2-ethylhexyl methacrylate, isobornyl methacrylate, methacrylic acid, benzyl methacrylate, phenyl methacrylate, methacrylonitrile, alpha-methylstyrene. methyl acrylate, ethyl acrylate, propyl acrylate (all isomers), butyl acrylate (all isomers), 2-ethylhexyl acrylate, isobornyl acrylate, acrylic acid, benzyl acrylate, phenyl acrylate, acrylonitrile. styrene, functional methacrylates, acrylates and styrenes selected from glycidyl methacrylate, 2-hydroxyethyl methacrylate, hydroxypropyl methacrylate (all isomers), hydroxybutyl methacrylate (all isomers), N,N-dimethylaminoethyl methacrylate, N,N-diethylaminoethyl methacrylate, triethyleneglycol methacrylate, itaconic anhydride, itaconic acid, glycidyl acrylate, 2-hydroxyethyl acrylate, hydroxypropyl acrylate (all isomers), hydroxybutyl acrylate (all isomers), N,N-dimethylaminoethyl acrylate, N,N-diethylaminoethyl acrylate, triethyleneglycol acrylate, methacrylamide, N-methylacrylamide, N,N-dimethylacrylamide, N-tert-butylmethacrylamide, N-n-butylmethacrylamide, N-methylolmethacrylamide, N-ethylolmethacrylamide. N-tert-butylacrylamide, N-n-butylacrylamide, N-methylolacrylamide, N-ethylolacrylamide, vinyl benzoic acid (all isomers), diethylaminostyrene (all isomers), alpha-methylvinyl benzoic acid (all isomers), diethylamino alpha-methylstyrene (all isomers), p-vinylbenzene sulfonic acid, p-vinylbenzene sulfonic sodium salt, trimethoxysilylpropyl methacrylate, triethoxysiiyipropyl methacrylate, tributoxysilylpropyl methacrylate, dimethoxymethylsilylpropyl methacrylate, diethoxymethyl-silylpropylmethacrylate, dibutoxymethylsilylpropyl methacrylate, diisopropoxymethylsilylpropyl methacrylate, dimethoxysilylpropyl methacrylate, diethoxysilylpropyl methacrylate, dibutoxysilylpropyl methacrylate, diisopropoxysilylpropyl methacrylate, trimethoxysilylpropyl acrylate, triethoxysiiyipropyl acrylate, tributoxysilylpropyl acrylate, dimethoxymethylsilylpropyl acrylate, diethoxymethylsilylpropyl acrylate, dibutoxymethylsilylpropyl acrylate, diisopropoxymethylsilylpropyl acrylate, dimethoxysilylpropyl acrylate, diethoxysilylpropyl acrylate, dibutoxysilylpropyl acrylate, diisopropoxysilylpropyl acrylate, vinyl acetate, vinyl butyrate, vinyl benzoate, vinyl chloride, vinyl fluoride, vinyl bromide, maleic anhydride, N-phenylmaleimide, N-butylmaleimide, N-vinylpyrrolidone, N-vinylcarbazole, butadiene, isoprene, chloroprene, propylene and mixtures thereof.

Preferred examples of vinylic monomers which comprise a hydroxyl group are selected from 2-hydroxyethyl-(meth)acrylate, all isomers of hydroxypropyl(meth)acrylates, including particularly 3-hydroxypropyl(meth)acrylate, and all isomers of butyl(meth)acrylates, including particularly 4-hydroxybutyl(meth)acrylate.

As usual in the field of methacrylate and acrylate compounds the notation "(meth)acrylate" is meant herein to refer to the corresponding acrylate and methacrylate derivatives as well.

The present invention can basically make use of any suitable method of generating free radicals such as the thermally induced homolytic scission of a suitable compound(s) (thermal initiators such as peroxides, peroxyesters, or azo compounds), the spontaneous generation from monomer (*e.g.*, styrene), redox initiating systems, photochemical initiating systems or high energy radiation such as electron beam, X- or gamma-radiation. The initiating system is chosen such that under the reaction conditions there is no substantial adverse interaction of the initiator or the initiating radicals with the transfer agent and the ROP partial system applied for introducing the polyester side into the graft polymers of the invention chains under the conditions of the experiment. The initiator should also have the requisite solubility in the reaction medium or monomer mixture. The initiators are used in conventional.

The use of thermal initiators is preferred. Said thermal initiators for the the polymerization of the one or more vinylic monomers are e.g. selected from 2,2'-azobis(isobutyronitrile), 2,2'-azobis(2-cyano-2-butane), dimethyl 2,2'-azobisdimethylisobutyrate, 4,4'-azobis(4-cyanopentanoic acid), 1,1'-azobis(cyclohexanecarbonitrile), 2-(t-butylazo)-2-cyanopropane, 2,2'-azobis[2-methyl-N-(1,1 )-bis(hydoxymethyl)-2-hydroxyethyl]propionamide, 2,2'-azobis[2-methyl-N-hydroxyethyl)]-propionamide, 2,2'-azobis(N,N'-dimethyleneisobutyramidine)dihydrochloride, 2,2'-azobis(2-amidinopropane)dihydrochloride, 2,2'-azobis(N,N'-dimethyleneisobutyramine), 2,2'-azobis(2-methyl-N-[1,1-bis(hydroxymethyl)-2-hydroxyethyl]propionamide), 2,2'-azobis(2-methyl-N-[1,1 -bis(hydroxymethyl)ethyl] propionamide), 2,2'-azobis[2-methyl-N-(2-hydroxyethyl) propionamide], 2,2'-azobis(isobutyramide)dihydrate, 2,2'-azobis(2,2,4-trimethylpentane), 2,2'-azobis(2-methylpropane), t-butyl peroxyacetate, t-butyl peroxybenzoate, t-butyl peroxyoctoate, t-butyl peroxyneodecanoate, t-butylperoxy isobutyrate, t-amyl peroxypivalate, t-butyl peroxypivalate, di-isopropyl peroxydicarbonate, dicyclohexyl peroxydicarbonate, dicumyl peroxide, dibenzoyl peroxide, dilauroyl peroxide, potassium peroxydisulfate, ammonium peroxydisulfate, di-t-butyl hyponitrite, dicumyl hyponitrite and mixtures thereof. 2,2'-azobis(isobutyronitrile) is a particularly preferred initiator. Thermal initiators are used at a initial concentration ranging between ($[RAFT]_0/20$) and ($[RAFT]_0/5$) preferably between ($[RAFT]_0/10$) and ($[RAFT]_0/5$), where ($[RAFT]_0$) is the initial concentration of the RAFT agent.

A process of the present invention can be applied to any cyclic ester compounds as grafting component as long as said cyclic ester is susceptible to a ring opening polymerisation in the given environment. Examples of suitable cyclic ester include in particular β-propiolactone; γ-butyrolactone, β-butyrolactone, δ-valerolactone, pivalolactone ε-caprolactone and glycolide, L-lactide, D-lactide, D,L-lactide and meso-lactide. Mixtures of such compounds can also be used where deemed appropriate.

Catalysts for the ring opening polymerization of said cyclic esters are known in the art and include in particular metal alkoxides, the metal of which is selected e.g. from Mg, Sn, Ti, Zr, Fe, Al, Y, Sm and Zn, like e.g. $Al(O\text{-}i\text{-}propyl)_3$; $Zn(O\text{-}n\text{-}propyl)_2$; $Ti(O\text{-}n\text{-}butyl)_4$; $(butyl)_3Sn(O\text{-}methyl)$; or $(butyl)_2Sn(O\text{-}methyl)_2$ and others, or Lewis acid compounds, particularly preferred, stannous 2-ethylhexanoate (stannous octoate). Said catalyst is used in catalytic amounts, ranging between 1 and 3 mol% of the cyclic ester and preferably around 1.5 mol% of the cyclic ester.

The molar ratio of different free-radically polymerizable monomers to be used for making a specific polymer is not critical, and can be chosen according to the requirements of the intended use of the polymer and properties of the graft polymers to be manufactured according to the present invention which appear mostly appropriate to the user.

The molar ratio of vinylic monomers comprising a hydroxyl group to other free-radically polymerizable monomers comprised in the reaction mixture can range from about 1:99 to about 100:0, preferably from 20:80 to 100:0, more preferably from 50:50 to 100:0, e.g. 80:20 to 100:0. Specific embodiments of the process of the present invention use e.g. reaction mixtures comprising a molar ratio of vinylic monomers comprising a hydroxyl group to said other free-radically polymerizable monomers ranging from 20:80 to 80:20, e.g. from 20:80 to 50:50 or from 50:50 to 80:20, thus leading to graft polymers according to the invention having a co-polymer-type of backbone wherein only a certain amount of the repeating units of the polymer is derived from a hydroxyl group containing monomer and thus grafted with a polyester side chains ("grafted repeating units"). Graft polymers comprising only grafted repeating units, either identical or different ones, can of course also be manufactured according to the invention. If desired for certain reasons, the process according to the invention can also be run in a way, which leaves a certain amount of unreacted (ungrafted)

hydroxyl groups in the polymer, although it is normally preferred to make fully polyester-grafted polymers.

The process according to the present invention is generally performed in the absence of oxygen or air and at a temperature being preferably about 95°C or higher, e.g. between 95°C, more preferably about 100°C, up to about 200°C.

The process according to the invention is generally carried out is an inert solvent, like e.g. benzene, toluene, THF, methylenechloride and the like. It may be necessary with certain specific reaction mixtures to try a few solvents in order to optimize the system, by the way of example, it has turned out that toluene is an exceptional solvent for use with a reaction system consisting of (a) 2-hydroxyethyl-(meth)acrylate; (b) 2-(2-cyanopropyl) dithiobenzoate; (c) ε-caprolactone; (d) stannous 2-octanoate; and (e) 2,2'-azobis(isobutyronitrile).

Another specifically preferred embodiment of the process of the invention is a process wherein the one or more vinylic monomers are selected from methyl (meth)acrylate, ethyl (meth)acrylate, propyl (meth)acrylates(all isomers), butyl methacrylates, phenyl methacrylate, (meth)acrylonitrile, alpha-methylstyrene, styrene and 2-hydroxyethyl-(meth) acrylate.

In yet another specific embodiment of process 2-hydroxyethyl methacrylate is used as the only vinylic monomer.

Still another specific embodiment is the process of the invention, wherein the cyclic ester is selected from ε-caprolactone, L-lactide, D-lactide and D,L-lactide.

Also preferred are the process variants according to the invention wherein the thermal initiator for the polymerization of the one or more vinylic monomers is 2,2'-azobis(isobutyronitrile), as well as the variants wherein the thiocarbonylthio compound used as chain transfer agent is 2-(2-cyanopropyl) dithiobenzoate.

Particularly preferred is a process according to the invention. said process comprising reacting a mixture of

(a) 2-hydroxyethyl methacrylate;
(b) 2-(2-cyanopropyl) dithiobenzoate;
(c) cyclic ester selected from ε-caprolactone, L-lactide, D-lactide and D,L-lactide;
(d) stannous 2-octanoate ($Sn(Oct)_2$); and
(e) 2,2'-azobis(isobutyronitrile

and reacting said mixture in toluene at a temperature of more than 100°C during 15 to 24 hours under exclusion of oxygen to form the desired graft copolymer.

In case of using ε-caprolactone said process results in a graft polymer substantially of the formula:

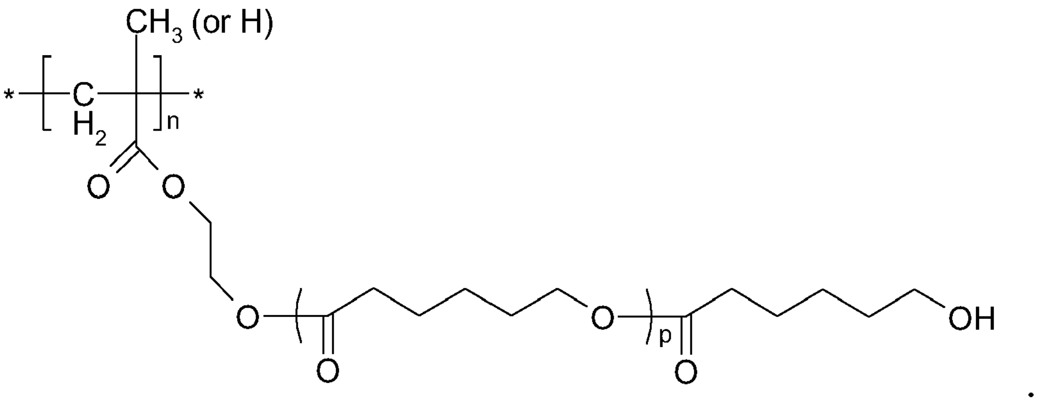

The index n in said formula ranges e.g. from about 5 to 200, in particular from 5 to 50, frequently from 5 to 15, the index p from about 1 to 1000, preferably from 1 to 100. A larger index p results normally in a better biocompatibility and/or degradebility. Both indices can be readily controlled, e.g. via the polymerization time. It may be advantageous in this regard to take samples after pre-selected time intervals.

The typical procedure for making a graft polymer according to the present invention is as follows: HEMA (2-hydroxyethyl methacrylate) as the vinylic monomer example (distilled over $CaH_2$ prior to use), CPDB (2-(2-cyanopropyl) dithiobenzoate), LA (L or D,L-lactide recrystallized three times from toluene) or CL (ε-caprolactone distilled under vacuum over $CaH_2$) and 60 mL of toluene (distilled over Na before use) were added to a 100 mL round-bottom flask. After having sealed the flask with a septum, the mixture solution is stirred with a magnetic stirrer, heated at 90 °C and degassed with $N_2$ for 30 minutes. Separately, stannous 2-octanoate (1.5 mol % / monomer) in solution in 1 mL of toluene and 2,2'-azobis-(isobutyronitrile) (AIBN; [AIBN] =[CPDB]/10) in solution in 0.5 mL of toluene are degassed at room temperature for 30 minutes. The AIBN and $Sn(Oct)_2$ solutions are then added to the reaction flask under $N_2$ via a canula. The temperature reaction is subsequently increased to 100 °C. When possible, aliquots are regularly taken during the reaction.

The reaction is stopped by exposing the mixture to air; the copolymer is recovered by precipitation into an excess of cold methanol or n-hexane, depending on the composition of copolymer. The obtained copolymer is finally dried under vacuum for 22 hours at room temperature.

In another aspect the present invention relates furthermore to the use of a polymer obtained according to the latter process as ingredient in pharmaceutical dosage forms, drug delivery systems, medical implants and in particular meshes or scaffolds for hernia repair and for abdominal wall repair.

A further subject of the invention is a graft polymer as described above, when obtained according to the process of the invention.

Example:

Short description of the drawings referring to the example

Fig.1 shows the RI/SEC trace of the purified PHEMA-*g*-PCL copolymer obtained after 22 hours of reaction time (SEC/DMAc).

Fig.2 represents the molar conversion of HEMA and CL versus time (gas chromatography).

Fig.3 shows the comparison of the RI and UV traces of the purified PHEMA-*g*-PCL copolymer (size exclusion chromatography in THF, 25 °C, 1 $mL.min^{-1}$, UV chromatogram recorded at 290 nm).

(A) Synthesis of 2-(2-cyanopropyl) dithiobenzoate according to the following reaction scheme:

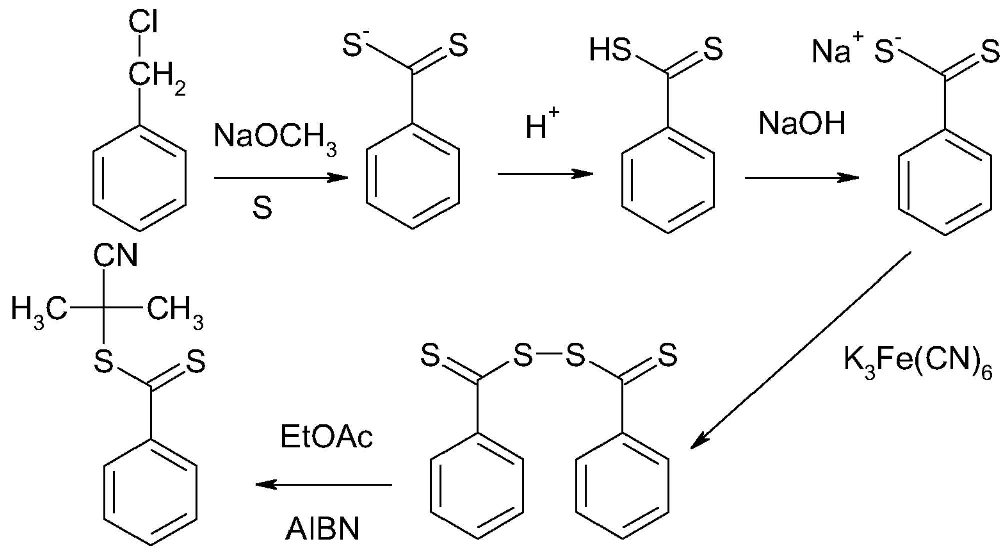


Benzyl chloride (12.6 g) is added drop-wise via an addition funnel over one hour to a round bottomed flask containing elemental sulfur (6.4 g), 25% sodium methoxide solution in methanol (40 g) and methanol (40 g). The resulting brown solution is then heated and allowed to reflux at 80°C overnight. After cooling to room temperature, the mixture is filtered to remove the white solid (sodium chloride) and then the methanol removed via rotary evaporation. The resulting brown solid is re-dissolved in distilled water (100 ml), and washed three times with diethyl ether (3 lots of 50 ml). A final layer of ether (50 ml) is added to the solution and the two phase mixture is then acidified with 32% aqueous HCl until the aqueous layer loses its characteristic brown color and the top layer is deep purple. The etherous (purple, dithiobenzoic acid) layer is extracted, before deionised water (120 ml) and 1.0 N NaOH (240 ml) are added to extract Sodium Dithiobenzoate as the aqueous layer. This washing process is repeated two more times to lead to a final solution of Sodium dithiobenzoate. Potassium ferricyanide (13.17g, 0.04mol) is dissolved in deionised water (200 ml). Sodium Dithiobenzoate solution (140 ml) is transferred to a conical flask equipped with a magnetic stirrer bar. Potassium ferricyanide solution is then added dropwise to the sodium dithiobenzoate via an addition funnel over a period of 1 hour under vigorous stirring. The red precipitate is filtered and washed with deionised water until the washings become colorless. The solid

is dried in vacuo at room temperature overnight. The product is recrystallised from ethanol. A solution of AIBN (5.84g, 0.021mol) and the recrystallised bis(thiobenzoyl) disulfide (4.25g, 0.014 mol) in ethyl acetate (80 ml) is heated at 80°C at reflux for 18 h. After removal of the volatiles in vacuo the crude product is subjected to silica gel column chromatography with ethyl acetate : n-hexane (2:3) as eluent to afford 2-Cyanoprop-2-yl Dithiobenzoate as red liquid. On keeping frozen overnight (-20°C), the product turned into a red solid.

(B) Manufacture of poly(2-hydroxyethyl methacrylate-graft- ε-caprolactone) (PHEMA-g-PCL graft copolymer):

0.16 g of HEMA (2-hydroxyethyl methacrylate, distilled over $CaH_2$ prior to use), 0.0088 g of CPDB (2-(2-cyanopropyl) dithiobenzoate), 9.8356 g of CL (ε-caprolactone distilled under vacuum over $CaH_2$) and 60 mL of toluene (distilled over Na before use) are added to a 100 mL round-bottom flask. After having sealed the flask with a septum, the mixture solution is then stirred with a magnetic stirrer, heated at 90 °C and degassed with $N_2$ for 30 minutes. Separately, $Sn(Oct)_2$ (1.5 mol % / monomer, e.g. 0.5236 g in the case of 9.8356 g of CL) in solution in 1 mL of toluene and 0.6 mg of AIBN (recrystallized from methanol, [AIBN] =[CPDB]/10) in solution in 0.5 mL of toluene are degassed at room temperature for 30 minutes. After degassing, the AIBN and $Sn(Oct)_2$ solutions are added to the reaction flask under $N_2$ via a canula. The reaction temperature is subsequently increased to 100 °C. When possible, aliquots are regularly taken during the reaction. The reaction is stopped by exposing it to air and the copolymer is recovered by precipitation in cold methanol. The obtained copolymer is finally dried under vacuum for 22 hours at room temperature.

The purified copolymer, after precipitation in cold methanol, was analyzed by SEC/DMAc. The apparent number average molecular weight, $M_n$, was 9000 g/mol and the polydispersity index, *PDI*, 1.20 (Fig. 1).

The conversion of both monomers is followed by gas chromatography (GC). After 22 hours at 100 °C, the molar conversion of HEMA and CL is respectively 80 % and 76 % (Fig. 2). It can be seen from said Figure that the consumption of CL always increases with time whereas the conversion of HEMA reaches 80 mol % within 1 hour. This observation in itself underpins the formation of a grafted copolymer.

The values of apparent number average molecular weight (Mn), polydispersity index (*PDI*) and molar conversions over the reaction time are presented in Table 1.

Table 1: Values of *Mn* and *PDI* obtained by SEC/DMAc and values of molar conversion of HEMA and CL measured by GC.

| *Time (h)* | *Time (min)* | *HEMA molar conversion* | *CL molar conversion* | *Average weight conversion* | *Mn (g/mol)* | *PDI* |
|---|---|---|---|---|---|---|
| 1 | 60 | 0.76 | 0.11 | 0.12 | 3000 | 1.10 |
| 2 | 120 | 0.81 | 0.26 | 0.26 | 4300 | 1.09 |
| 3 | 180 | 0.76 | 0.31 | 0.31 | 5200 | 1.09 |
| 4 | 240 | 0.57 | 0.19 | 0.20 | 6100 | 1.09 |
| 5 | 300 | 0.74 | 0.38 | 0.39 | 6800 | 1.07 |
| 6 | 360 | 0.72 | 0.44 | 0.45 | 7400 | 1.07 |
| 7 | 420 | 0.64 | 0.36 | 0.36 | 7500 | 1.10 |
| 8 | 480 | 0.62 | 0.43 | 0.43 | 8140 | 1.06 |
| 22 | 1320 | 0.80 | 0.76 | 0.76 | 9250 | 1.14 |

The UV and RI/SEC traces of the purified copolymer match together, that also attests the formation of a grafted copolymer (Fig.3). The UV spectrum was recorded at 290 nm, the wavelength corresponding to the C=S double bond present as end groups of the non-degradable polymer backbone.

The peaks at 6.25 and 5.90 ppm are attributed to the vinylic protons of HEMA and indicate that only a very little amount of polycaprolactone chains is initiated by HEMA and thus non-grafted to the backbone of PHEMA.

## Claims

1. A combined RAFT/ROP process for the preparation of a graft co-polymer having a backbone built-up from repeating units derived by polymerization of free-radically polymerizable monomers comprising vinylic monomers, and polyester side chains grafted to said backbone derived by ring-opening polymerisation of cyclic ester compounds; said

process comprising reacting a mixture of

(a) one or more vinylic monomers, optionally in combination with other monomers susceptible to free-radical polymerization, wherein at least one of said vinylic monomers comprises a (free) hydroxyl group;
(b) a thiocarbonylthio compound as chain transfer agent;
(c) one or more cyclic esters, which is/are susceptible to a ring-opening polymerization; and
(d) a compound effective for initiating the ring-opening polymerization of the one or more cyclic esters;

and reacting said mixture in an inert solvent at a temperature of more than 90°C under exclusion of oxygen to form the graft copolymer.

**2.** A process according to claim 1 wherein the thiocarbonylthio compound is a compound of formula

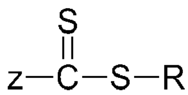

wherein
Z is selected from hydrogen, fluorine, chlorine, optionally substituted alkyl, optionally substituted aryl. optionally substituted heterocyclyl, optionally substituted alkylthio, - COOR", -COOH, -$O_2$CR", -CONR"$_2$), -CN, -P(=0)OR"$_2$, and - P(=0)R"$_2$]-, wherein R" is selected from optionally substituted $C_1$-$C_{18}$ alkyl, $C_2$-$C_{18}$ alkenyl, aryl, heterocyclyl, aralkyl, alkaryl wherein the substituents are independently selected from the group that consists of epoxy, hydroxy, alkoxy, acyl, acyloxy. carboxy (and salts), sulfonic acid (and salts), alkoxy- or aryloxy-carbonyl, isocyanato, cyano, silyl. halo, and dialkylamino; and
R is selected from optionally substituted alkyl; an optionally substituted saturated, unsaturated or aromatic carbocyclic or heterocyclic ring; optionally substituted alkylthio; optionally substituted alkoxy; and optionally substituted dialkylamino.

**3.** A process according to anyone of claims 1 or 2, wherein the vinylic monomers are selected from formula

$CH_2$=CUV

wherein
U is selected from hydrogen, halogen, optionally substituted $C_1$-$C_4$alkyl wherein the substituents are independently selected from hydroxy, $C_1$-$C_{18}$ alkoxy, aryloxy, carboxy, -$O_2$CR", $C_1$-$C_{18}$ alkoxycarbonyl and aryloxycarbonyl; and V is selected from hydrogen, R", $CO_2$H, $CO_2$R", COR" , CN, $CONH_2$, CONHR", CONR$^M{}_2$, $O_2$CR", OR" and halogen, wherein R" has the same meaning as in claim 2.

**4.** A process according to any one of claims 1 to 3, wherein the vinylic monomer which comprises a hydroxyl group is selected from 2-hydroxyethyl(meth)acrylate, 4-hydroxybutyl(meth)acrylate hydroxypropyl(meth)acrylates, including particularly 3-hydroxypropyl(meth)acrylate, and butyl(meth)acrylates, including particularly 2-hydroxyethyl methacrylate.

**5.** A process according to any one of claim 1 to 4; wherein the cyclic ester is selected from β-propiolactone; γ-butyrolactone, β-butyrolactone, δ-valerolactone, pivalolactone ε-caprolactone and glycolide, L-lactide, D-lactide, D,L-lactide and meso-lactide.

**6.** A process according to any one of claims 1 to 5, wherein the compound effective for initiating the ring-opening polymerization of the one or more cyclic esters is selected from metal alkoxides, said metal being selected from Mg, Sn, Ti, Zr, Fe, Al, Y, Sm and Zn, or stannous 2-ethylhexanoate (stannous 2-octanoate).

**7.** A process according to any one of claims 1 to 6, wherein the reaction mixture furthermore comprisse

(e) a thermal initiator for the the polymerization of the one or more vinylic monomers.

**8.** A process according to any one of claim 1 to 7, wherein the reaction tempearture is from about 95°C, preferably

about 100°C, to about 200°C.

9. A process according to any one of claims 1 to 8, wherein the inert solvent is selected from benzene, toluene, THF and methylenechloride.

10. A process according to anyone of claims 1 to 9, wherein the one or more vinylic monomers are selected from methyl methacrylate, ethyl methacrylate, propyl methacrylate (all isomers), butyl methacrylate (all isomers), 2-ethylhexyl methacrylate, isobornyl methacrylate, methacrylic acid, benzyl methacrylate, phenyl methacrylate, methacrylonitrile, alpha-methylstyrene. methyl acrylate, ethyl acrylate, propyl acrylate (all isomers), butyl acrylate (all isomers), 2-ethylhexyl acrylate, isobornyl acrylate, acrylic acid, benzyl acrylate, phenyl acrylate, acrylonitrile. styrene, functional methacrylates, acrylates and styrenes selected from glycidyl methacrylate, 2-hydroxyethyl methacrylate, hydroxypropyl methacrylate (all isomers), hydroxybutyl methacrylate (all isomers), N,N-dimethylaminoethyl methacrylate, N,N-diethylaminoethyl methacrylate, triethyleneglycol methacrylate, itaconic anhydride, itaconic acid, glycidyl acrylate, 2-hydroxyethyl acrylate, hydroxypropyl acrylate (all isomers), hydroxybutyl acrylate (all isomers), N,N-dimethylaminoethyl acrylate, N,N-diethylaminoethyl acrylate, triethyleneglycol acrylate, methacrylamide, N-methylacrylamide, N,N-dimethylacrylamide, N-tert-butylmethacrylamide, N-n-butylmethacrylamide, N-methylolmethacrylamide, N-ethylolmethacrylamide. N-tert-butylacrylamide, N-n-butylacrylamide, N-methylolacrylamide, N-ethylolacrylamide, vinyl benzoic acid (all isomers), diethylaminostyrene (all isomers), alpha-methylvinyl benzoic acid (all isomers), diethylamino alpha-methylstyrene (all isomers), p-vinylbenzene sulfonic acid, p-vinylbenzene sulfonic sodium salt, trimethoxysilylpropyl methacrylate, triethoxysiiyipropyl methacrylate, tributoxysilylpropyl methacrylate, dimethoxymethylsilylpropyl methacrylate, diethoxymethyl-silylpropylmethacrylate, dibutoxymethylsilylpropyl methacrylate, diisopropoxymethylsilylpropyl methacrylate, dimethoxysilylpropyl methacrylate, diethoxysilylpropyl methacrylate, dibutoxysilylpropyl methacrylate, diisopropoxysilylpropyl methacrylate, trimethoxysilylpropyl acrylate, triethoxysiiyipropyl acrylate, tributoxysilylpropyl acrylate, dimethoxymethylsilylpropyl acrylate, diethoxymethylsilylpropyl acrylate, dibutoxymethylsilylpropyl acrylate, diisopropoxymethylsilylpropyl acrylate, dimethoxysilylpropyl acrylate, diethoxysilylpropyl acrylate, dibutoxysilylpropyl acrylate, diisopropoxysilylpropyl acrylate, vinyl acetate, vinyl butyrate, vinyl benzoate, vinyl chloride, vinyl fluoride, vinyl bromide, maleic anhydride, N-phenylmaleimide, N-butylmaleimide, N-vinylpyrrolidone, N-vinylcarbazole, butadiene, isoprene, chloroprene, propylene and mixtures thereof.

11. A process according to any one of claims 7 to 10, wherein the thermal initiator for the the polymerization of the one or more vinylic monomers is selected from 2,2'-azobis(isobutyronitrile), 2,2'-azobis(2-cyano-2-butane), dimethyl 2,2'-azobisdimethylisobutyrate, 4,4'-azobis(4-cyanopentanoic acid), 1,1'-azobis(cyclohexanecarbonitrile), 2-(t-butylazo)-2-cyanopropane, 2,2'-azobis[2-methyl-N-(1,1 )-bis(hydoxymethyl)-2-hydroxyethyl]propionamide, 2,2'-azobis[2-methyl-N-hydroxyethyl)]-propionamide, 2,2'-azobis(N,N'-dimethyleneisobutyramidine)dihydrochloride, 2,2'-azobis(2-amidinopropane)dihydrochloride, 2,2'-azobis(N,N'-dimethyleneisobutyramine), 2,2'-azobis(2-methyl-N-[1,1-bis(hydroxymethyl)-2-hydroxyethyl]propionamide), 2,2'-azobis(2-methyl-N-[1,1 -bis(hydroxymethyl)ethyl] propionamide), 2,2'-azobis[2-methyl-N-(2-hydroxyethyl) propionamide], 2,2'-azobis(isobutyramide)dihydrate, 2,2'-azobis(2,2,4-trimethylpentane), 2,2'-azobis(2-methylpropane), t-butyl peroxyacetate, t-butyl peroxybenzoate, t-butyl peroxyoctoate, t-butyl peroxyneodecanoate, t-butylperoxy isobutyrate, t-amyl peroxypivalate, t-butyl peroxypivalate, di-isopropyl peroxydicarbonate, dicyclohexyl peroxydicarbonate, dicumyl peroxide, dibenzoyl peroxide, dilauroyl peroxide, potassium peroxydisulfate, ammonium peroxydisulfate, di-t-butyl hyponitrite, dicumyl hyponitrite and mixtures thereof.

12. A process according to anyone of claims 1 to 11, wherein the thiocarbonylthio compound used as chain transfer agent is selected from dithiobenzoic acid benzyl ester; dithiobenzoic acid 1-phenyl-ethyl ester; dithiobenzoic acid 1-methyl-1-phenyl-ethyl ester; acetic acid 1-thiobenzoylsulfanyl-ethyl ester; dithiobenzoic acid 1-(4-methoxyphenyl)-ethyl ester; thiobenzoylsulfanyl-acetic acid ethyl ester; 2-methyl-2- thiobenzoylsulfanyl-propionic acid ethyl ester; dithiobenzoic acid tert.-butyl ester; dithiobenzoic acid cyano-dimethyl-methyl ester (=2-(2-cyanopropyl) dithiobenzoate); dithiobenzoic acid 1,1,3,3-tetramethyl-butyl ester; dithiobenzoic acid 1-(4-chlorophenyl)-1-methyl-ethyl ester; 4-chloro-dithiobenzoic acid 1-methyl-1-phenyl-ethyl ester; naphthalen-1-carbodithionic acid 1-methyl-1-phenyl-ethyl ester and 4-cyano-4-methyl-4-thiobenzoylsulfanyl-butyric acid.

13. A process according to any one of claims 1 to 12, wherein the one or more vinylic monomers are selected from methyl (meth)acrylate, ethyl (meth)acrylate, propyl (meth)acrylates(all isomers), butyl methacrylates, phenyl methacrylate, (meth)acrylonitrile, alpha-methylstyrene, styrene and 2-hydroxyethyl(meth)acrylate.

14. A process according to claim 13, wherein 2-hydroxyethyl methacrylate is used as the only vinylic monomer.

**15.** A process according to any one of claim 1 to 14, wherein the cyclic ester is selected from ε-caprolactone, L-lactide, D-lactide and D,L-lactide.

**16.** A process according to anyone of claims 1 to 15, wherein the thermal initiator for the polymerization of the one or more vinylic monomers is selected from 2,2'-azobis(isobutyronitrile).

**17.** A process according to anyone of claims 1 to 16, wherein the thiocarbonylthio compound used as chain transfer agent is 2-(2-cyanopropyl) dithiobenzoate.

**18.** A process according to anyone of claims 1 to 17. said process comprising reacting a mixture of

(a) 2-hydroxyethyl methacrylate;
(b) 2-(2-cyanopropyl) dithiobenzoate;
(c) cyclic ester selected from ε-caprolactone, L-lactide, D-lactide and D,L-lactide;
(d) stannous 2-octanoate; and
(e) 2,2'-azobis(isobutyronitrile)

and reacting said mixture in toluene at a temperature of more than 100°C during 15 to 24 hours under exclusion of oxygen to form the graft copolymer consisting of repeating units of the formula:

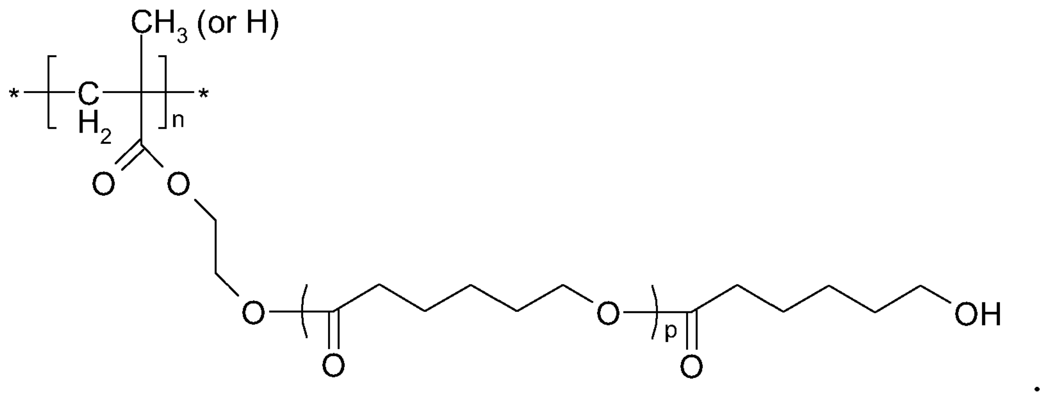

**19.** A process according to anyone of claims 1 to 18, which is performed in one pot.

**20.** A composition comprising

(a) one or more vinylic monomers, optionally in combination with other monomers susceptible to free-radical polymerization, wherein at least one of said vinylic monomers comprises a (free) hydroxyl group;
(b) a thiocarbonylthio compound as chain transfer agent;
(c) one or more cyclic esters, which is/are susceptible to a ring-opening polymerization; and
(d) a compound effective for initiating the ring-opening polymerization of the one or more cyclic esters.

**21.** A graft polymer when obtained according to anyone of claims 1 to 19.

**22.** Use of a polymer obtained according to anyone of claims 1 to 18, in particular according to claim 18, as ingredient in pharmaceutical dosage forms, drug delivery systems, medical implants and in particular meshes or scaffolds for hernia repair and for abdominal wall repair.

Fig.1: RI/SEC trace of the purified copolymer obtained after 22 hours of reaction time (SEC/DMAc)

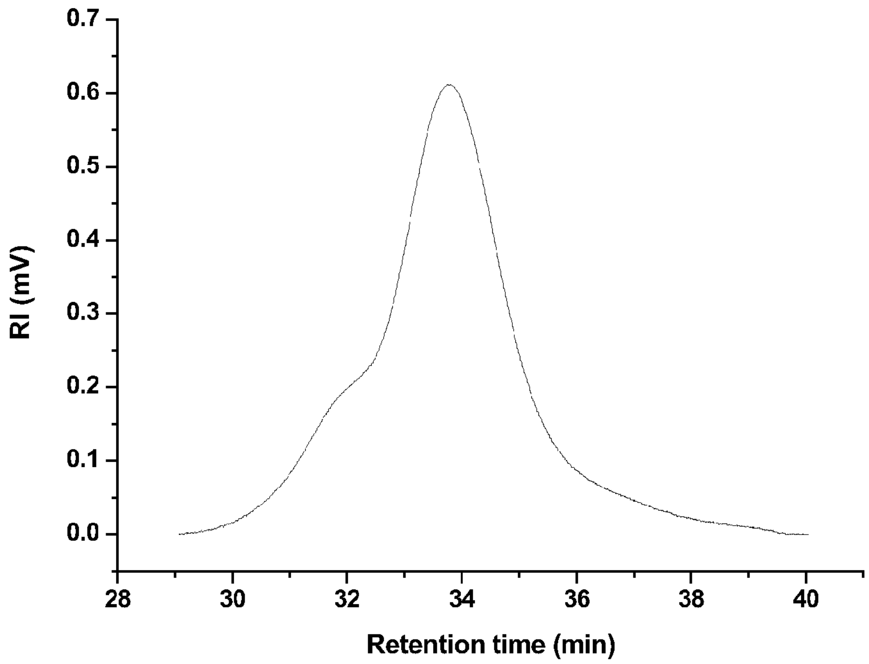


Fig.2: Molar conversion of HEMA and CL versus time (gas chromatography)

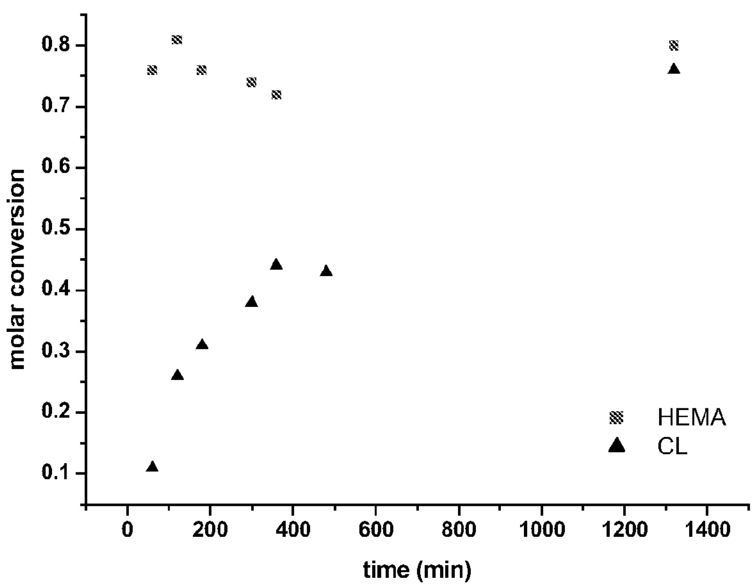

Fig.3 . Comparison of the RI and UV traces of the purified PHEMA-*g*-PCL copolymer (size exclusion chromatography in THF, 25 °C, 1 $mL.min^{-1}$, UV chromatogram recorded at 290 nm).

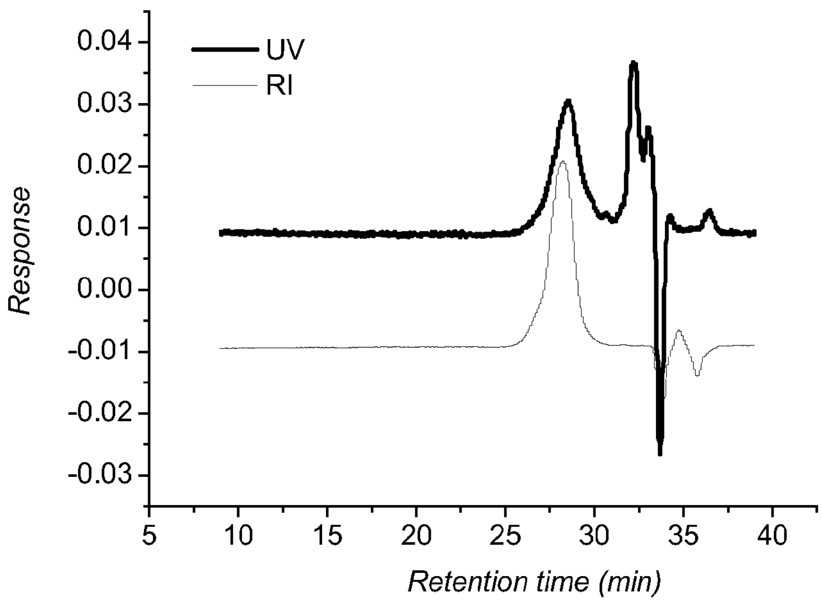

European Patent Office

# EUROPEAN SEARCH REPORT

Application Number
EP 07 10 1223

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br>A | VILLARROYA S ET AL: "Synthesis of Graft Copolymers by the Combination of ATRP and Enzymatic ROP in scCO2" MACROMOLECULES, vol. 39, 2006, pages 9080-9086, XP002436421<br>* the whole document * | 21,22<br><br><br><br><br><br>1-10,18, 20 | INV. C08F291/08 C08F265/04 C08F2/38<br><br>ADD. A61K9/00 A61L27/14 A61L31/04 |
| X | WO 2005/054318 A (UNIV UTRECHT [NL]; HENNINK WILHELMUS EVERHARDUS [NL]; VAN NOSTRUM CORN) 16 June 2005 (2005-06-16)<br>* page 16, line 25 - line 26 *<br>* example 4 *<br>* claims 10,12,15,16,24 * | 21,22 | |
| X | LIM D W ET AL: "A NEW CLASS OF BIODEGRADABLE HYDROGELS STEREOCOMPLEXES BY ENANTIOMERIC OLIGO(LACTIDE) SIDE CHAINS OF POLY(HEMA-g-OLA)S" MACROMOLECULAR: RAPID COMMUNICATIONS, WILEY VCH VERLAG, WEINHEIM, DE, vol. 21, no. 8, 2000, pages 464-471, XP002323307 ISSN: 1022-1336<br>* page 471 *<br>* Conclusion * | 21,22 | TECHNICAL FIELDS SEARCHED (IPC)<br><br>C08F<br>A61K<br>A61L |
| X | JAKUBOWSKI, WOJCIECH ET AL: "New segmented copolymers by combination of atom transfer radical polymerization and ring opening polymerization" MACROMOLECULAR SYMPOSIA , 240(RECENT TRENDS IN IONIC POLYMERIZATION), 213-223 CODEN: MSYMEC; ISSN: 1022-1360, 2006, XP002436422<br>* Scheme 4 *<br>* page 221 *<br><br>-/-- | 21 | |

~~The present search report has been drawn up for all claims~~

| Place of search | Date of completion of the search | Examiner |
|---|---|---|
| Munich | 28 June 2007 | Friebe, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

6

EPO FORM 1503 03.82 (P04C01)

European Patent Office

# EUROPEAN SEARCH REPORT

Application Number
EP 07 10 1223

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | XU XUEWEI ET AL: "Synthesis and characterization of well-defined poly(2-hydroxyethyl methacrylate-co-styrene)-graft-poly(epsilon -caprolactone) by sequential controlled polymerization" J POLYM SCI PART A; JOURNAL OF POLYMER SCIENCE, PART A: POLYMER CHEMISTRY NOV 1 2004, vol. 42, no. 21, 1 November 2004 (2004-11-01), pages 5523-5529, XP002436423 | 21 | |
| A | * the whole document * | 1-10,18, 20 | |
| A | WO 2004/056880 A (STICHTING DUTCH POLYMER INST [NL]; VAN BENTHEM-VAN DUUREN ANNA MA [NL]) 8 July 2004 (2004-07-08) * example IV * * claim 5 * | 1-10,18, 20 | |
| | | | TECHNICAL FIELDS SEARCHED (IPC) |

~~The present search report has been drawn up for all claims~~

| Place of search | Date of completion of the search | Examiner |
|---|---|---|
| Munich | 28 June 2007 | Friebe, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

6
EPO FORM 1503 03.82 (P04C01)

European Patent Office

Application Number

EP 07 10 1223

## CLAIMS INCURRING FEES

The present European patent application comprised at the time of filing more than ten claims.

- [X] Only part of the claims have been paid within the prescribed time limit. The present European search report has been drawn up for the first ten claims and for those claims for which claims fees have been paid, namely claim(s):

  18,20,21,22

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

## ANNEX TO THE EUROPEAN SEARCH REPORT ON EUROPEAN PATENT APPLICATION NO.

EP 07 10 1223

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2007

| Patent document cited in search report | Publication date | Patent family member(s) | Publication date |
|---|---|---|---|
| WO 2005054318 A | 16-06-2005 | NONE | |
| WO 2004056880 A | 08-07-2004 | AU 2003295256 A1 | 14-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9801478 A

### Non-patent literature cited in the description

- **X. XU ; J. HUANG.** Synthesis and Characterization of Well-Defined Poly(2-hydroxyethyl methacrylate-co-styrene)-graft-poly(ε-caprolactone) by Sequential Controlled Polymerization. *Journal of Polymer Science Part A: Polymer Chemistry,* 2004, vol. 42, 5523.5529